## Europäisches Patentamt

## European Patent Office

## Office européen des brevets

(19)

(11) Veröffentlichungsnummer: **0 068 446**
**B2**

(12) **NEUE EUROPÄISCHE PATENTSCHRIFT**

(45) Veröffentlichungstag der neuen Patentschrift:
**07.12.88**

(51) Int. Cl.⁴: **A 61 K 9/22**, A 01 N 25/00,
C 05 G 3/00

(21) Anmeldenummer: **82105557.1**

(22) Anmeldetag: **24.06.82**

(54) Verfahren zur zeitlichen Steuerung der Freisetzung von Wirkstoffen aus Wirkstoffzubereitungen, insbesondere Arzneimittelzubereitungen.

(30) Priorität: **26.06.81 DE 3125178**

(43) Veröffentlichungstag der Anmeldung:
**05.01.83 Patentblatt 83/1**

(45) Bekanntmachung des Hinweises auf die Patenterteilung:
**18.12.85 Patentblatt 85/51**

(45) Bekanntmachung des Hinweises auf die Entscheidung über den Einspruch:
**07.12.88 Patentblatt 88/49**

(84) Benannte Vertragsstaaten:
**AT BE CH DE FR GB IT LI LU NL SE**

(73) Patentinhaber: **GÖDECKE AKTIENGESELLSCHAFT, Salzufer 16, D-1000 Berlin 10 (DE)**

(72) Erfinder: **Knecht, Adolf, Dr., Kapellenweg 4a, D-7800 Freiburg/Brsg (DE)**
Erfinder: **Augart, Helmut, Tannenweg 28a, D-7808 Waldkirch (DE)**

(56) Entgegenhaltungen:
**CHEMICAL ABSTRACTS, Band 79, Nr. 11, 17. September 1973, Seite 25, Nr. 61534e, Columbus, Ohio, USA Z. KOROSSYOVA et al.: "Effect of surface-active and hydrophilic macromolecular adjuvants on the resorption of drugs from drug forms"**
**Journal of Pharmaceutical Sciences, Band 55, (1966), pp. 974-976**
**Pharmaceutica Acta Helvetica, Band 55, (1980), pp. 189-197**

**Die Akte enthält technische Angaben, die nach dem Eingang der Anmeldung eingereicht wurden und die nicht in dieser Patentschrift enthalten sind.**

## Beschreibung

In der pharmazeutischen Technik ist es bekannt. Wirkstoffe so in Arzneimittelzubereitungen einzuarbeiten, dass die Wirkstoffe aus diesen Zubereitungen mit einer vorgegebenen Geschwindigkeit freigegeben werden. Hierdurch kann beispielsweise eine Verlängerung der Wirkdauer erzielt und damit eine zu schnelle und/oder zu konzentrierte Anflutung des Wirkstoffs und zu hohe und damit zu Nebenwirkungen führende Spitzen der Blut- bzw. Gewebespiegel vermieden werden.

Ist die gute Löslichkeit oder bei peroralen pharmazeutischen Produkten auch die gute Resorbierbarkeit des Wirkstoffes der Grund für eine zu schnelle Anflutung, dann kann ein solcher Wirkstoff durch Behinderung der ursprünglichen Lösungseigenschaften hinsichtlich der Resorption retardiert werden. Im einfachsten Falle geschieht dies z.B. bei gut löslichen Substanzen durch Zumischen von schlecht löslichen Hilfsstoffen oder durch Überziehen der Substanz mit Hilfsstoffen, die die Löslichkeit herabsetzen. Ganz allgemein gilt die Regel, dass gut wasserlösliche Substanzen mit schlecht löslichen Hilfsstoffen und schlecht wasserlösliche Substanzen mit gut löslichen bzw. gut quellbaren Hilfsstoffen verarbeitet werden müssen, wenn eine gute Retardierung erhalten werden soll. Oft werden solche Zubereitungen sogar zusätzlich mit Diffusionshüllen umgeben.

Es ist auch bekannt, dass hydrophile Polymere für die Retardierung eingesetzt werden.

Aus J. Pharm. Sci. 55 S. 974 (1966) ist z.B. bekannt, dass die angestrebte Retardierung ausschliesslich auf der Bildung einer viskosen Gel-Barriere beruht, die das Eindringen von Verdauungssäften in das Tabletteninnere verhindert.

Im übrigen wurden hydrophile Polymere in der pharmazeutischen Technik bisher nur als Bindemittel in wässriger Lösung zur Erzielung mechanisch stabiler Komprimate und solche mit hoher Quellfähigkeit als sogenannte Sprengmittel zur Erzielung eines raschen Tablettenzerfalls in fester Form eingesetzt.

Für den Fachmann war es bisher sehr schwierig, beim Einsatz von Quellstoffen, wie z.B. Guargum, Johannisbrotkernmehl, halbsynthetischen oder synthetischen Polymeren, wie Kieselsäuren, Celluloseacetatphthalat, Hydroxypropylcellulose oder Carbopol deren Einfluss auf die Freigabe des Wirkstoffs aus einem Arzneimittel vorauszusehen und es bedurfte bisher erst recht umfangreicher empirischer Versuche, um Retardformen mit einer vorher festgelegten Freigabecharakteristik des Wirkstoffs zu entwickeln.

Aufgabe der vorliegenden Erfindung ist es, durch Anwendung einer bisher nicht bekannten Gesetzmässigkeit die Freigabecharakteristik von Retardformen mit einfachsten Mitteln in beliebiger Weise einzustellen und somit die empirischen Versuche bei der Entwicklung auf ein Minimum zu reduzieren.

Es wurde überraschend gefunden, dass beim Einsatz von hydrophilen Polymeren, insbesondere von Carboxymethylcellulose und Methylcellulose, die – etwa durch den Substitutionsgrad bedingten – Unterschiede der Löslichkeit oder Quellbarkeit nur einen recht geringen Einfluss auf die Wirkstofffreisetzung von retardierten Arzneimittelformen haben.

Der Fachmann hätte aufgrund seiner Kenntnisse des Standes der Technik eigentlich erwarten müssen, dass bei der Einarbeitung von beispielsweise Carboxymethylcellulose in Retard-Zubereitungen der Substitutionsgrad für die Aberosion von Komprimaten und die damit verbundene Wirkstofffreisetzung von wesentlicher Bedeutung ist, da die Löslichkeit und Quellbarkeit dieses Hilfsstoffs unmittelbar vom Substitutionsgrad abhängt, so dass bei Kontakt mit Wasser oder den Verdauungssäften durch unterschiedlich starke Quellung und Auflockerung der Arzneimittelzubereitungen auch eine stark unterschiedliche Freisetzungsrate resultieren müsste. Völlig unerwartet wurde gefunden, dass die Freisetzung des Wirkstoffs im wesentlichen nur durch den Viskositätsgrad der zugegebenen hydrophilen Polymeren bestimmt wird, wobei wiederum völlig überraschend mit hochviskosen Polymeren die Einstellung einer weniger verzögerten Wirkstofffreisetzung gelingt, wohingegen mit niedrigviskosen Polymeren die Wirkstofffreisetzung stärker verzögert wird.

Gegenstand der Erfindung ist demnach ein Verfahren zur zeitlichen Steuerung der Freisetzung von Wirkstoffen aus retardierten Arzneimittelzubereitungen, dadurch gekennzeichnet, dass man hydrophile Polymere in einer Menge von 0,1 bis 10 Gew.% zugibt, wobei die mit steigenden Viskositätsstufen des zugegebenen hydrophilen Polymeren zunehmende Freigabegeschwindigkeit empirisch ermittelt und die gewünschte Freigabecharakteristik anhand der ermittelten Werte eingestellt wird.

Besonders bevorzugte hydrophile Polymere sind Carboxymethylcellulosen (Synonyme: Natriumcarboxymethylcellulose, CMC, Na-Celluloseglycolat). Diese sind in grosser Vielfalt auf dem Markt. Sie unterscheiden sich einerseits durch den Substitutionsgrad, andererseits innerhalb bestimmter Substitutionsbereiche durch die Viskosität, die ihrerseits vom Polymerisationsgrad bzw. vom Molekulargewicht abhängig ist. Die Viskosität der üblichen Handelsprodukte wird in wässriger Lösung bestimmt und vom Hersteller typenspezifisch angegeben. Die Angaben der Patentanmeldung beziehen sich auf diese von den Herstellern angegebenen Daten und beruhen auf der Viskositätsbestimmung, veröffentlicht in der Firmenschrift Technische Daten Cellulose Gum, chemische und physik. Eigenschaften 800-6A/G der Firma Hercules Wilmington/Delaware USA.

Nicht nur durch den Einsatz von hydrophilen Polymeren einer ganz bestimmten Viskosität, sondern auch durch Mischung von Polymeren verschiedener Viskositätsgrade kann nunmehr die Freisetzungsrate von Wirkstoffen aus festen Retardzubereitungen in bisher nicht bekannter Weise variiert und festgelegt werden. Bei der Verwendung einer gründlichen Mischung von beispielsweise Carboxymethylcellulose unterschiedlicher Viskosität erhält man Retardprodukte, deren Freisetzungsverlauf zwischen den Freisetzungsverläufen liegt, die die einzelnen Komponenten ergeben hätten.

Carboxymethylcellulosen eignen sich insbesondere deshalb besonders gut für das erfindungsgemässe Verfahren, weil diese in einer Vielzahl von Viskositätsabstufungen von ca. 0,02 Pa.s. bis ca. 40 Pa.s. im Handel sind und insbesondere hinsichtlich ihrer Stabilität und Verträglichkeit mit Wirkstoffen und anderen Hilfsstoffen keinerlei Probleme aufwerfen.

Das erfindungsgemässe Verfahren ist vorteilhaft für die Entwicklung von Retard-Arzneimitteln, da eine grosse Anzahl von Versuchen eingespart werden kann. Bisher war es erforderlich, in grossen Versuchsreihen Zusammensetzungen zu finden, die die erforderliche Wirkstoff-Liberation ergaben. Dabei wurden Rezepturen entweder willkürlich ausgewählt und in Versuchsreihen variiert oder aber es wurden nach einem Faktorenversuchsplan bestimmte einflussreiche Faktoren ausgesucht, die jeweils auf verschiedenem Niveau variiert werden mussten. Dabei mussten die geplanten Versuche durchgeführt, die erhaltenen Produkte untersucht und aufgrund der Ergebnisse die Auswahl für die nächste Versuchsreihe getroffen werden.

Bei Einsatz des erfindungsgemässen Verfahrens sind diese Arbeiten in der Regel nicht erforderlich oder wesentlich im Umfang reduziert; vielmehr kann nach einem Vorversuch die voraussichtlich geeignete Viskositätsstufe von beispielsweise Carboxymethylcellulose ausgesucht werden. Ergibt sich dabei nicht die gewünschte Wirkstoffliberation, dann kann durch die Verwendung von Mischungen von Carboxymethylcellulose mit verschiedenen Viskositätsgraden eine geeignete Freisetzung eingestellt werden.

Ist diese Einstellung erfolgt, dann resultieren Produkte, die jeweils in ihrer qualitativen und quantitativen Zusammensetzung gleich sind und sich nur durch den Viskositätsgrad der eingesetzten Carboxymethylcellulosen unterscheiden.

Da die Produkte gleich zusammengesetzt sind, sind auch die Verarbeitungseigenschaften gleich. Es muss also nicht befürchtet werden, dass sich bei der Variierung der Freisetzungsrate wie bisher auch die physikalischen Eigenschaften, wie beispielsweise die Tablettierbarkeit, ändern. Auch die Wirkstoff-Stabilität solcher Produkte ist für alle Viskositätsstufen gleich und muss daher nicht mehr in lang dauernden Stabilitätsprüfungen für jede Variation getrennt ermittelt werden.

Die Mengen des zugesetzten Polymeren liegen in der Regel zwischen 0,1 und 10 Gew.% der gesamten Masse der Arzneimittelzubereitung. Bei Verwendung von Carboxymethylcellulose sind Mengen im Bereich von 0,5–3 Gew.% bevorzugt.

Das Verfahren kann grundsätzlich auch für andere Wirkstoffzubereitungen, wie z.B. Tierköder, Düngemittel oder Herbizide angewandt werden.

Die nachfolgenden Beispiele dienen der näheren Erläuterung der Ausführung des Verfahrens:

Beispiel 1

100 g hydriertes Rizinusöl, 150 g Stearinsäure und 750 g Lactose wurden mit 10 g Carboxymethylcellulose verschiedener Viskositätsstufen homogen gemischt und in an sich bekannter Weise schmelzgranuliert.

Zum Einsatz kamen folgende Viskositätsstufen:

a)  0,05  Pa.s.
b)  0,3   Pa.s.
c)  0,6   Pa.s.
d)  3     Pa.s.
e)  6     Pa.s.
f)  20    Pa.s.
g)  40    Pa.s.

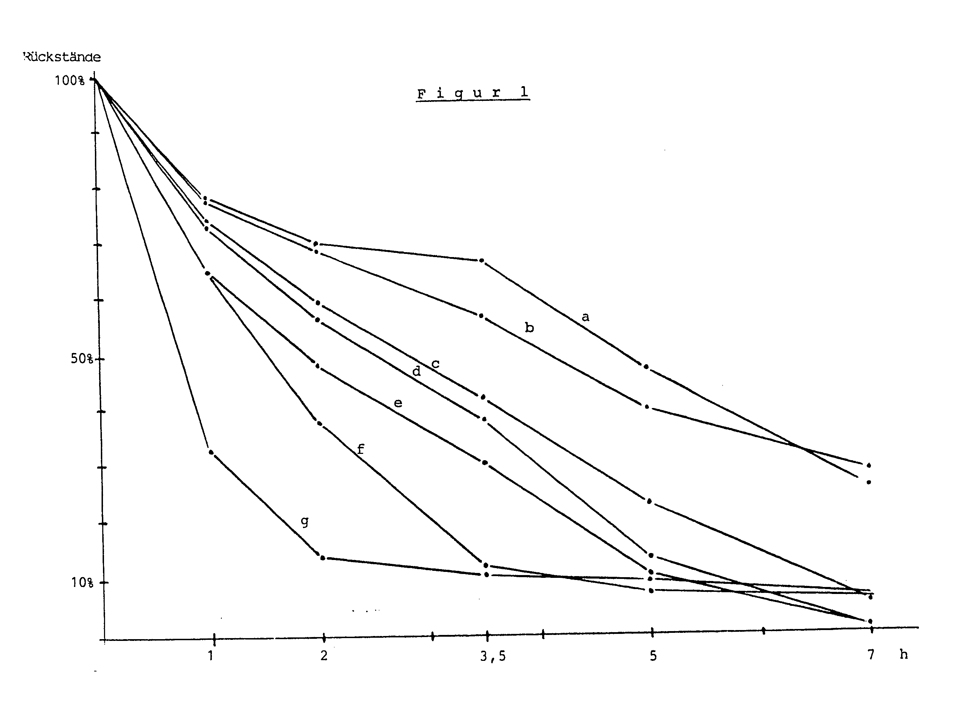

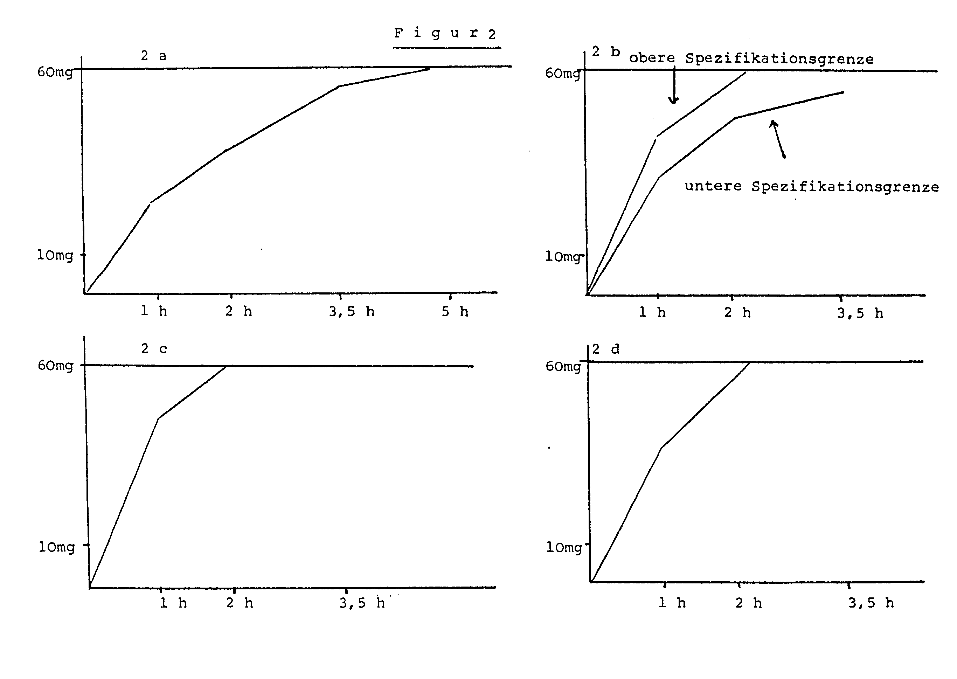

Alle Ansätze wurden dann bei mittlerem gleichem Druck zu runden gewölbten Tabletten mit 11 mm Durchmesser und 490 mg Gewicht verpresst. Der Zerfall der Tabletten wurde nach National Formulary XIV, S. 985, in künstlichem Magen- und Darmsaft auf deren Gewichtsverlust untersucht, wobei die Rückstände der Tabletten gemäss Fig. 1 im Abstand gemäss Abszisse entnommen, bei 45°C 10 Stunden getrocknet und gewogen wurden.

Aus Fig. 1 ergibt sich deutlich, dass der Gewichtsverlust der Tabletten in unmittelbarer Abhängigkeit von der Viskosität der zugesetzten Carboxymethylcellulose erfolgt.

Beispiel 2

Es soll die Einstellung einer gezielten Wirkstoffreigabe anhand einer vorgegebenen Spezifikation demonstriert werden.

Gemäss Beispiel 1 werden Tabletten mit einem Gesamtgewicht von 300 mg und mit einem Wirkstoffgehalt von 60 mg (Diltiazem-HCl) hergestellt. Die Bestimmung des Wirkstoffs erfolgte im Abstand gemäss Abszisse aus dem flüssigen Medium. Zunächst wurde eine Carboxymethylcellulose mit einer Viskosität von 0,3 Pa.s. zugesetzt.

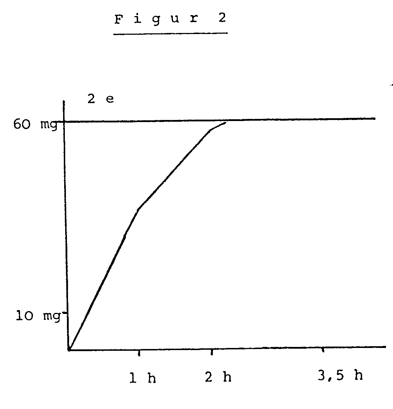

Das Ergebnis ist in Fig. 2a wiedergegeben.

Vorgegeben waren Freigabegrenzen gemäss Fig. 2b.

Aufgrund der Kurvenläufe in Fig. 1 wurde nunmehr eine unter Verwendung von Carboxymethylcellulose der Viskosität 3 Pa.s. hergestellte Tablette untersucht. Das Ergebnis der Freisetzungsprüfung ist in Fig. 2c wiedergegeben und zeigt, dass die Freisetzung zu schnell erfolgte. Die Verwendung einer Carboxymethylcellulose mit 0,6 Pa.s. ergab dann eine genau innerhalb der vorgelegten Grenzen liegende Freisetzungscharakteristik (Fig. 2d). Dasselbe Ergebnis wurde durch Mischung einer Carboxymethylcellulose von 0,3 Pa.s. mit einer solchen von 3,0 Pa.s. erreicht (vgl. Fig. 2e).

**Patentansprüche**

1. Verfahren zur zeitlichen Steuerung der Freigabe von Wirkstoffen aus mittels schlecht löslichen Hilfsstoffen retardierten Wirkstoffzubereitungen, insbesondere Arzneimittelzubereitungen, dadurch gekennzeichnet, dass man hydrophile Polymere in einer Menge von 0,1 bis 10 Gew.% zugibt, wobei die mit steigenden Viskositätsstufen der zugegebenen hydrophilen Polymeren zunehmende Freigabegeschwindigkeit empirisch ermittelt und die gewünschte Freigabecharakteristik anhand der ermittelten Werte eingestellt wird.

2. Verfahren gemäss Anspruch 1, dadurch gekennzeichnet, dass als hydrophile Polymere Methylcellulose und/oder Carboxymethylcellulose zugegeben werden.

3. Verfahren gemäss Anspruch 1 und 2, dadurch gekennzeichnet, dass die Viskosität des hydrophilen Polymeren im Bereich von 0,02 bis 40 Pa.s. liegt.

4. Verfahren gemäss Anspruch 1 bis 3, dadurch gekennzeichnet, dass man die Viskosität durch Mischung von hydrophilen Polymeren verschiedener Viskositätsgrade auf einen zwischen diesen Graden liegenden Wert einstellt.

**Claims**

1. Process for the time controlled release of active substances form sustained-release active substance preparations, in particular drug preparations, characterized in that hydrophilic polymers are added in an amount of 0,1 to 10 weight % while the release rate which is rising as a result of increasing viscosity steps of the hydrophilic polymers added is empirically determined and the desired release characteristic is adjusted using the values determined.

2. Process according to claim 1 characterized in that as polymers hydrophilic methylcellulose and/or carboxymethylcellulose are added.

3. Process according to claims 1 and 2 characterized in that the viscosity of the hydrophilic polymer is within the range of 0.02 to 40 Pa.s.

4. Process according to claims 1 to 3, characterized in that the viscosity is adjusted by mixing hydrophilic polymers of different viscosity degrees to obtain a value between these degrees.

**Revendications**

1. Procédé de contrôle dans le temps de la libération d'agents actifs à partir de préparations renfermant ceux-ci, notamment à partir de préparations pharmaceutiques, caractérisé en ce qu'on ajoute des polymères hydrophiles dans une quantité de 0,1 à 10% (poids) en déterminant empiriquement la vitesse de libération qui croît lorsque le degré de viscosité du polymère hydrophile ajouté augmente, après quoi l'on ajuste la caractéristique de libération voulue, sur la base des valeurs ainsi déterminées.

2. Procédé selon la revendication 1, caractérisé en ce qu'on ajoute, en tant que polymères hydrophiles, de la méthylcellulose et/ou de la carboxyméthylcellulose.

3. Procédé selon les revendications 1 et 2, caractérisé en ce que la viscosité du polymère hydrophile est comprise entre 0,02 et 40 Pa.s.

4. Procédé selon les revendications 1 à 3, caractérisé en ce qu'on mélange des polymères hydrophiles présentant des degrés de viscosité différents pour ajuster la viscosité à une valuer située entre lesdits degrés.

Figur 1

F i g u r 2

2 a

60mg

10mg

1 h   2 h   3,5 h   5 h

2 b  obere Spezifikationsgrenze

60mg

10mg

untere Spezifikationsgrenze

1 h   2 h   3,5 h

2 c

60mg

10mg

1 h   2 h   3,5 h

2 d

60mg

10mg

1 h   2 h   3,5 h

F i g u r   2